# EUROPEAN PATENT APPLICATION

(11) **EP 0 646 375 A1**
(43) Date of publication of application: **05.04.1995**
(21) Application number: 94115571.5
(22) Date of filing: 04.10.1994
(51) Int. Cl.: A61K 38/05

(54) **Method of regulating blood amino acid levels**

(30) Priority: 05.10.1993 US 132500
(71) Applicant: CLINTEC NUTRITION COMPANY, Deerfield, Illinois 60015 (US)
(72) Inventor: Madsen, David C., Libertyville, IL 60048 (US); Rowe, Bruce W., Evanston, IL 60202 (US); Smith, Ross C., Level 2, Royal North Shore Hospital, Sydney (AU)
(74) Representative: Vuille, Roman

(57) **Abstract**

The use of peptides for manufacturing a composition for parenterally administering amino acids to a patient. The composition provides amino acids to the patient in the form of peptides. The structure of the peptides are chosen so as to provide a desired clearance of the amino acids from the blood. Preferably, the peptides are dipeptides.

## Description

The present invention relates generally to the parenteral infusion of compositions into a patient. More specifically the present invention relates to the infusion of amino acids into a patient.

Of course, it is known in the medical arts to enterally and parenterally infuse compositions into a patient for a variety of reasons. Such compositions can include medicaments, as well as nutriments such as amino acids.

The resultant blood level of any composition that is infused, ingested, or injected into a patient, is a function of: its infusion rate; its rate of clearance (CL) from the vascular compartments; its rate of entry into a vascular compartment; and its rate of endogenous generation. When administering compositions, achieving a given blood level may be critical for the given therapeutic application. One can modify the blood level of the composition that is infused by either altering the infusion rate or the concentration of the composition.

Amino acids are, of course, an essential component of the nutritional requirements of a patient. For many medical conditions, it may be necessary to parenterally provide amino acids to a patient. Although numerous compositions are commercially available that contain amino acids, for at least certain applications traditional amino acid solutions may not function entirely satisfactorily.

Current intravenous nutrition (IVN) with amino acids may be limited in its ability to provide at least tyrosine, cysteine and glutamine as free amino acids, which may be essential for patients requiring intravenous nutrition. Thus interest has been raised in utilizing the dipeptide forms of at least some amino acids as substrates.

Dipeptides provide methods for providing amino acids that have been difficult to administer in their free amino acid form. See U.S. Patent Nos. 5,122,515 and 5,189,016. In the blood stream, the peptides are hydrolysed into their constituent amino acids. These amino acids are then taken up and utilized by the tissue of the patient.

The dipeptide forms of, for example, tyrosine, glutamine, and cysteine are generally more stable and soluble than the free forms. This characteristic of peptides may facilitate the parenteral infusion of the constituent amino acids into a patient. Greater solubility allows more concentrated solutions to be administered to a patient, which reduces fluid input.

Dipeptides would favor peripheral vein intravenous nutrition infusion because they allow the infusion of twice as much amino acid per osmole compared to free amino acids. While the metabolism and physiology of dipeptides is well-studied, little is known about the intravascular fate of amino acids resulting from dipeptide breakdown.

When amino acids are infused as peptides, one can modify the blood levels of the amino acids by either altering the infusion rate or the concentration of the peptides that function as the source of the amino acids. However, it is not possible to modify the clearance of the amino acids directly, since this is a pharmacological property of each compound. Additionally, it is only by means of considerable nutrient manipulation that one can modify the rate of endogenous generation of amino acids; for example, by changing the total dose of protein or of calories.

Blood levels of amino acids are important for a number of reasons including toxicity concerns. Such concerns are especially great when the solution is for use in neonates. In many neonates the amino acids tyrosine, glycine, and phenylalanine can cause toxicity problems due to in-born errors of metabolism. Therefore, it is necessary to maintain blood levels of these amino acids in such neonates at sufficiently low levels.

Heretofore, it has been suggested in the art, that one can modify blood levels of amino acids, released from peptides, only by changing the concentration, or rate of infusion, of each peptide in the infusate. Additionally, it was believed that one could influence clearance by choosing a peptide with a faster/slower rate of clearance based on the assumption that the clearance of the constituent amino acid was directly related to, and predictable from, the clearance of the parent peptide. But, in contrast to the state of the art, Applicants have found that the clearance of a given amino acid varies with the parent peptide. Based on this discovery, Applicants have devised methods of regulating blood levels of amino acids.

It has been surprisingly discovered that the rate of clearance of a given amino acid is not directly related to the clearance of the parent peptide. Therefore, it has been surprisingly discovered, that one can alter the clearance of an amino acid by choosing a specific parent peptide, but not necessarily based on the clearance of that peptide.

The present invention provides for the use of peptides for manufacturing a composition for administering desired amino acids to a patient in the form of peptides. The structure of the peptides are chosen so as to provide a desired clearance of the constituent amino acids from the blood.

In an embodiment the peptides are dipeptides.

Additionally, the present invention provides a method for determining the clearance of amino acids that are administered parenterally into a patient via peptides. The method comprises the steps of: preparing a composition that includes peptides that are to provide amino acids upon hydrolysis in a patient's blood stream or by hydrolysis on the external surface of cells; and relating the clearance of the amino acids from the blood stream to the structure of the peptide that is infused into the patient's blood stream.

Further, the present invention provides for the use of peptides for manufacturing a composition for regulating blood concentrations of amino acids that are administered parenterally to a patient. To this end, amino acids are administered to a patient in the form of peptides, the structure of the peptides is chosen so as to provide the desired clearance of the amino acids.

Moreover, in an embodiment, the present invention provides a composition for safely providing amino acids to neonates.

An advantage of the present invention is to provide a composition for regulating blood levels of amino acids.

Furthermore, an advantage of the present invention is that it provides a composition and method for allowing one to select specific parent peptides to modulate the final blood levels of the constituent amino acids.

A still further advantage of the present invention is to provide a composition and method for increasing blood concentrations of specific amino acids which heretofore were not possible due to toxicity concerns.

Moreover, another advantage of the present invention is to provide a composition and method for increasing blood levels of amino acids without incurring prohibitive costs.

Further, an advantage of the present invention is to provide a composition and method for safely delivering amino acids such as tyrosine, phenylalanine, and glycine to a neonate.

Another advantage of the present invention is to provide a composition and method for increasing blood levels of amino acids where heretofore stability was an issue.

Additional features and advantages of the present invention are described in, and will be apparent from, the detailed description of the presently preferred embodiments and from the drawings.

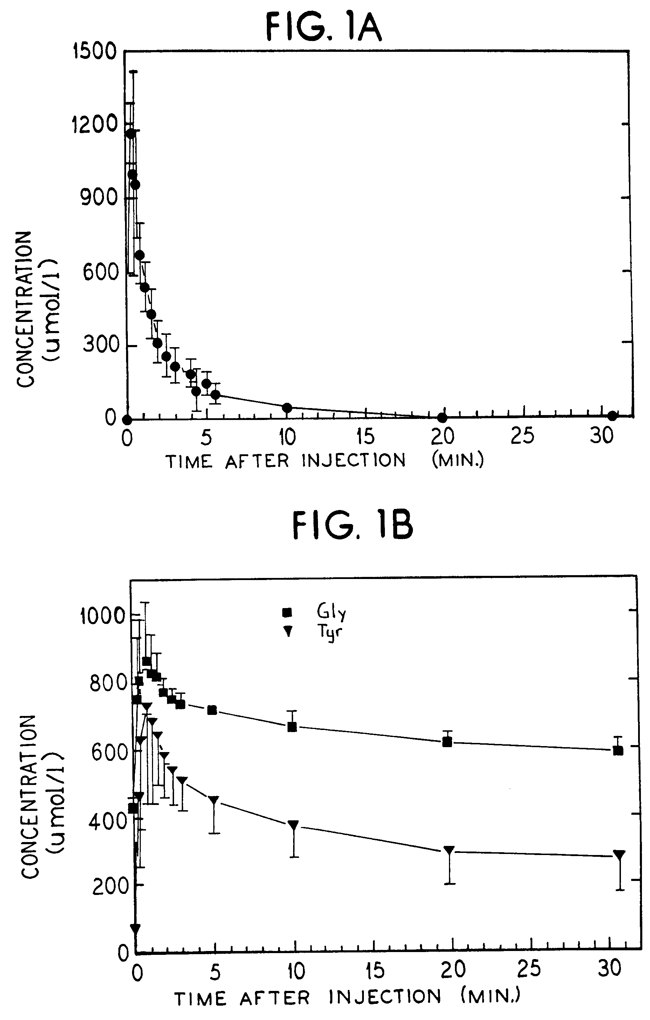

Figure 1 illustrates blood levels for amino acids over time for dipeptides and constituent amino acids following a bolus injection of dipeptide.

Specifically, Figure 1A illustrates the concentration over time of GlyTyr.

Figure 1B illustrates the concentration over time of the constituent amino acids glycine and tyrosine.

Figure 2 illustrates dipeptide clearance for a number of dipeptides.

Figure 3 illustrates volume of distribution (V_{B}) of certain dipeptides.

Figure 4 illustrates the terminal half-lifes of certain dipeptides.

Figure 5 illustrates kinetic parameters for glutamine liberated from various dipeptides.

Figure 6 illustrates kinetic parameters for glycine liberated from dipeptide breakdown.

Figure 7 illustrates kinetic parameters for alanine liberated from dipeptide breakdown.

Figure 8 illustrates kinetic parameters for tyrosine liberated from dipeptide breakdown.

It has been surprisingly found that in contrast to the belief in the state of the art, heretofore, that there is no correlation between the rate of clearance (CL) of a given dipeptide and the constituent amino acids. Indeed, there is a statistically significant difference in the clearance of the same amino acids derived from different dipeptides, i.e., dipeptides having the same amino acids, but in a different structure.

It has been surprisingly found that the clearance of dipeptides from the blood is a function of the structure of the dipeptide. It has also been found that the release of amino acids from the dipeptides is more rapid than the clearance from the blood. The clearance of the amino acids is a function of the structure of the parent dipeptide. Accordingly, one can alter the clearance of an amino acid by choosing a parent peptide having a specific structure.

Therefore, by judicious selection of a parent peptide one can modulate the final blood levels of the constituent amino acids. This thereby allows one to select a peptide which has the particular properties necessary for a specific use: cost; stability; clearance (and thereby blood levels) of the peptides; and clearance (and thereby blood levels) of amino acids.

The present invention thereby provides for the use of peptides for manufacturing a composition for administering amino acids to a patient. To this end, the present invention provides for parenterally administering amino acids to a patient by administering the desired amino acids to the patient in the form of a peptide and choosing the structure of the peptides so as to provide a desired clearance of the constituent amino acids from the blood.

Additionally, using the composition of the present invention, a method for determining the clearance of amino acids that are administered parenterally into a patient via peptides is provided. The method comprises the steps of: preparing a composition that includes peptides that are to provide amino acids upon hydrolysis in a patient's blood stream or on the external surface of cells; determining the clearance of the amino acids from the blood steam; and relating clearance to the structure of the peptide that is infused into the patient's blood stream.

Further, the present invention provides a method for regulating blood concentrations of amino acids that are administered parenterally to a patient. The method comprises the steps of: administering amino acids to a patient in the form of peptides; and choosing the structure of the peptides so as to provide the desired clearance of the amino acids.

The advantages of the present invention can be achieved by first determining the rate of clearance of each peptide and its amino acids. In certain situations, it may be desirable to achieve a higher or lower level of a specific amino acid which is administered in the form of a peptide. Pursuant to the present invention, one can alter the blood concentration not only by increasing/decreasing infusion rate of the peptide, or its concentration in the infusate, but also by choosing the structure of the peptide to confer on its constituent amino acid a desired clearance.

Several dipeptides will be referred to in the following discussions, in abbreviated form. Table I lists the abbreviations and the chemicals to which they refer.

**TABLE I**

| DIPEPTIDE ABBREVIATION | DIPEPTIDE STRUCTURE | |
|---|---|---|
| | N-terminal | C-terminal |
| 1. "GLN-ALA" | Glutamine | Alanine |
| 2. "ALA-GLN" | Alanine | Glutamine |
| 3. "GLN-GLY" | Glutamine | Glycine |
| 4. "GLY-GLN" | Glycine | Glutamine |
| 5. "GLY-TYR" | Glycine | Tyrosine |
| 6. "TYR-ALA" | Tyrosine | Alanine |
| 7. "ALA-TYR" | Alanine | Tyrosine |

For example, one may employ a parenteral solution with GlyGln as the source of glutamine. In order to increase the blood concentration of glutamine, one can increase the concentration of GlyGln. However, it may not be desirable to do so if the toxicity of this dipeptide - or of glycine - would then be unacceptably high. Likewise, if the cost of the dipeptide were prohibitive or if stability were a problem with respect to the dipeptide, this could make it difficult, if not impossible, to increase the blood concentration by merely increasing the concentration of dipeptide.

Since the blood level of any entity is inversely related to its clearance, one can consider either using, pursuant to the present invention, GlnGly or AlaGly, in place of GlyGln. The faster clearance of these two dipeptides, means that they disappear from the bloodstream more rapidly than does GlyGln. However, since the clearance of Gln derived from GlnGly, or from AlaGln, is slower than is found for GlyGln, this also means that higher blood levels of Gln will be achieved, but without increasing the concentration or infusion rate of GlnGly or AlaGln.

By way of example, and not limitation, an example of the present invention will not be set forth:

### Example:

### Study Protocol

Experiments were performed on mature male Sprague Dawley rats (see Table II for weights). Prior to the experiments the animals were maintained on standard rat chow (Doust and Rabbidge, Sydney, Australia) and tap water. The animals were starved for 15-18h and then anaesthetized by peritoneal injection with Nembutal (Boehringer Ingelheim, Sydney, Australia) (4mg/kg Body Weight (B.W.) pentobarbitone). The internal jugular veins were exposed by blunt dissection and a small gauge silicone catheter (Dow Corning, Sydney, Australia) was introduced and secured with 5-0 silk sutures. Both internal jugular veins were catheterized; the left for dipeptide injection and the right for blood sampling. After insertion, the catheters were kept patent and with occasional flushes (0.2-0.5ml) of heparinized (10U/ml) isotonic saline.

All dipeptides were proven by to be >98% pure. TyrGly was not studied due to is very low solubility. Dipeptides were dissolved in 80mM sodium phosphate buffer, Ph 7.0, made isotonic with sodium chloride and filtered through a 0.2µm membrane filter (Millipore, Sydney, Australia).

Following an initial baseline blood sample (0.15-0.2ml) a bolus injection of dipeptide (0.2ml, see Table 1 for doses) was administered into the left catheter. Rapid blood samples were taken in the first 5 minutes and then at 10, 20 and 30 minutes following injection. Heparinized (10U/ml) isotonic saline (0.5ml) was injected into the sample catheter after the 5, 10 and 20 minute blood samples to maintain patency of the catheter. At each time point two blood samples were taken: the first, 0.1ml in volume, to remove traces of heparinized saline and blood from the catheter and the second, 0.15-ml in volume, for dipeptide and amino acid determination. One hour after injection of the dipeptide the bladder was exposed and urine was collected with a small gauge hypodermic needle.

**TABLE II**

| **Characteristics of Animals** | | |
|---|---|---|
| Dipeptide | Body Weight (g) | Dipeptide Dose (µmol/100g BW) |
| Gly Tyr | 409 ± 24.1 | 21.0 |
| Ala Tyr | 381 ± 23.6 | 46.0 |
| Tyr Ala | 399 ± 36.4 | 46.0 |
| Gln Gly | 376 ± 20.0 | 46.0 |
| Gly Gln | 414 ± 48.9 | 46.0 |
| Ala Gln | 373 ± 27.1 | 46.0 |
| Gln Ala | 399 ± 38.5 | 15.5 |
| Note: Values are mean ± S.D. for 5 animals for each dipeptide. TyrGly was not studied to very low solubility. | | |

### Analysis

Immediately following collection the blood (0.15-ml) and urine (0.5ml) samples were transferred to pre-weighed stoppered tubes containing 0.6ml of ice-cold 4% (w/v) sulfosalicylic acid. The samples were shaken vigorously and placed on ice for 10 minutes to allow complete deproteinization. The sample tubes were weighed and then centrifuged (bench-top, 1200 x g) for 10 minutes at 2-4°C. The resulting supernatant was filtered (0.45µm membrane, Millipore, Sydney, Australia) and stored at - 80°C. The volume of blood added to the tubes was determined by weight assuming a blood density of 1.06g/ml.

The concentration of dipeptides and amino acids in the deproteinized samples was determined using a HPLC modification of the standard ion-exchange chromatography, post-column ninhydrin method for physiological amino acids. The column temperature was modified for each dipeptide to produce complete separation of dipeptides and amino acids. The column temperatures were: 70°C for AlaGln, GlyTyr and GlnGly, 60°C for GlyGln, 55°C for GlnAla, and 45°C for AlaTyr. The coefficient of variation for the measurement of amino acids and dipeptides in whole blood was 1-4% and the recovery of amino acids and dipeptides following complete sample preparation was 98-102%.

### Data Analysis

Kinetic parameters were calculated for the disappearance of dipeptides, and the amino acids released from the dipeptides using standard equations. In calculating the amino acid clearance the dose was assumed to be that administered as the dipeptide. Baseline amino acid concentrations were subtracted from the amino acid concentrations measured following dipeptide injection to obtain the AUC for amino acids released from the dipeptides. AUC for dipeptides and the constituent amino acids was calculated using the trapezoidal method with extrapolation to infinite time.

Blood clearance was calculated by dividing the dose of dipeptide by the area under the blood concentration-versus-time curve (AUC). Elimination half-lifes (t_{½}) were estimated from the relationship: t_{½} = ln2/β, where β is the rate constant for the terminal elimination phase. β was obtained by fitting the concentration-versus-time-curves to a two-compartment model by non-linear least squares regression. Apparent volume of distribution (V_{β}) for the dipeptides was calculated using the formula: V_{β} for the amino acids was calculated by dividing clearance by β. The kinetic parameters were calculated for each animal individually and the results for each dipeptide were then averaged.

Initially comparisons of kinetic parameters for the dipeptides and the same amino acid from different dipeptides were made using analysis of variance (ANOVA). If significant differences were detected by ANOVA, comparisons between individual dipeptides and amino acids were performed using Tukey's multiple range test. Comparisons of kinetic parameters of the amino acids released from the same dipeptide and between amino acids and their parent dipeptides were performed using paired t-tests. Differences were considered statistically significant when P<0.05.

### RESULTS

The blood concentration of GlyTyr and its constituents glycine and tyrosine following intravenous injection of GlyTyr are presented in Figure 1. The concentration profiles of the other dipeptides and their constituents are qualitatively similar and thus are not shown. The dipeptide concentration fell rapidly following injection reaching undetectable levels 20 minutes after injection.

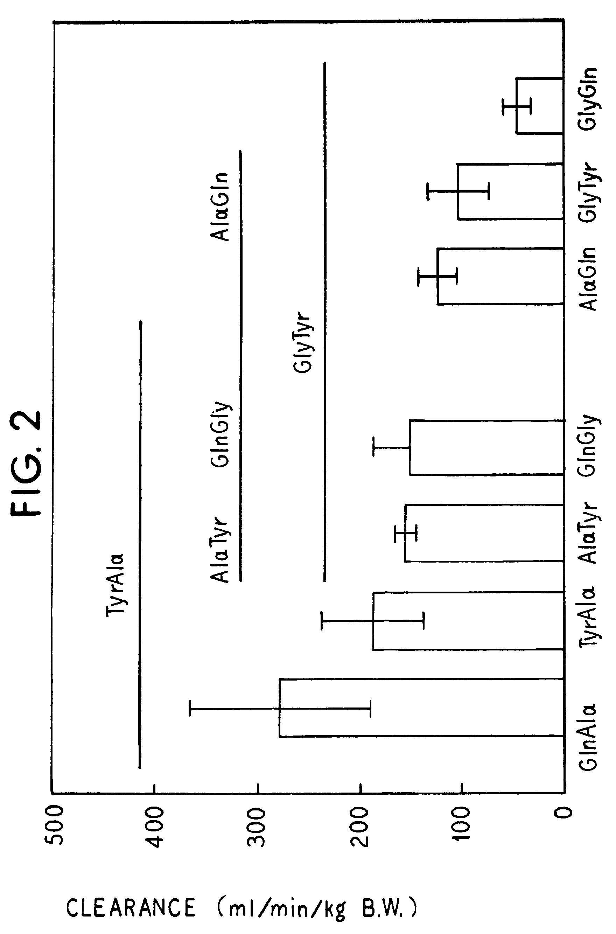

The fall in dipeptide concentration was accompanied by a large and rapid increase in the concentration of the constituent amino acids (see Figure 1B). The amino acid concentrations reached a maximum at 1.0 to 2.0 minutes following dipeptide injection and thereafter fell slowly. The levels of most amino acids were still above baseline 30 minutes after injection.
*Kinetics of Dipeptide Disappearance from Blood (Table III and Figures 2-4).*

The mean clearances of the dipeptides (Table III) differed (P<0.001). Glutaminyl-dipeptides with the small neutral amino acids glycine and alanine in the C-terminal position (GlnGly and GlnAla) had greater (P<0.01) mean clearance than the respective N-terminal glycyl- or alanyl-dipeptide isomers (GlyGln and AlaGln) (see Figure 2). The mean clearances of the N-terminal alanyl-dipeptides (AlaTyr and AlaGln) were greater than the respective N-terminal glycyl-dipeptides (GlyTyr and GlyGln) (see Figure 2).

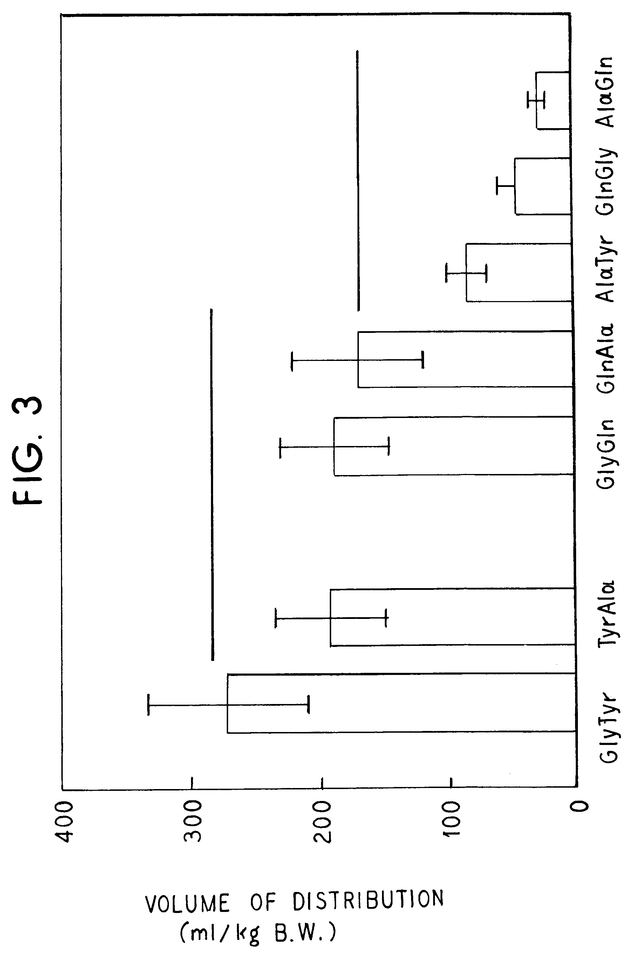

There was considerable variability in the apparent volume of distribution (V_{β}) of the dipeptides (P<0.001) (see Figure 3). For many dipeptides (GlyTyr, TyrAla, GlyGln and GlnAla) the mean V_{β} was larger than the blood volume of rats (60-70 ml/kg) indicating either significant distribution in the non-vascular pools and/or binding the tissue components. As was the case for clearance there were significant differences in the mean V_{β} between the various glutaminyl- and tyrosyl-dipeptide isomers (see Figure 3).

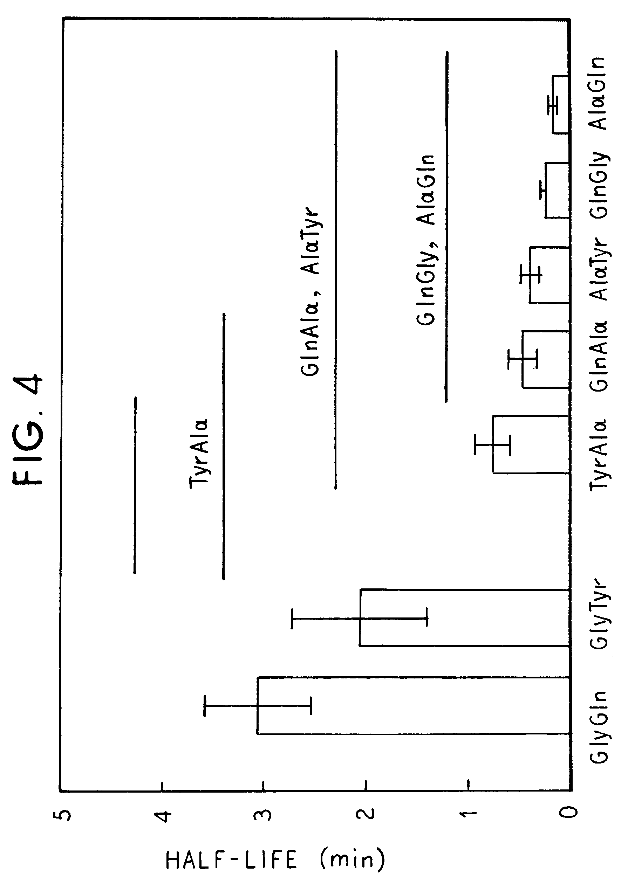

The relatively short half-lives of the dipeptides shown in Figure 4 reflects the rapid decline in dipeptide concentration following intravenous injection (see Figure 1A). In general there was an inverse relationship between t_{½} and clearance (compare Figures 2 and 4). However, because t_{½} and V_{β} (GlyTyr and AlaGln) had similar clearance values.
*Kinetics of Amino Acid Disappearance following Dipeptide Breakdown (Tables III and IV).*

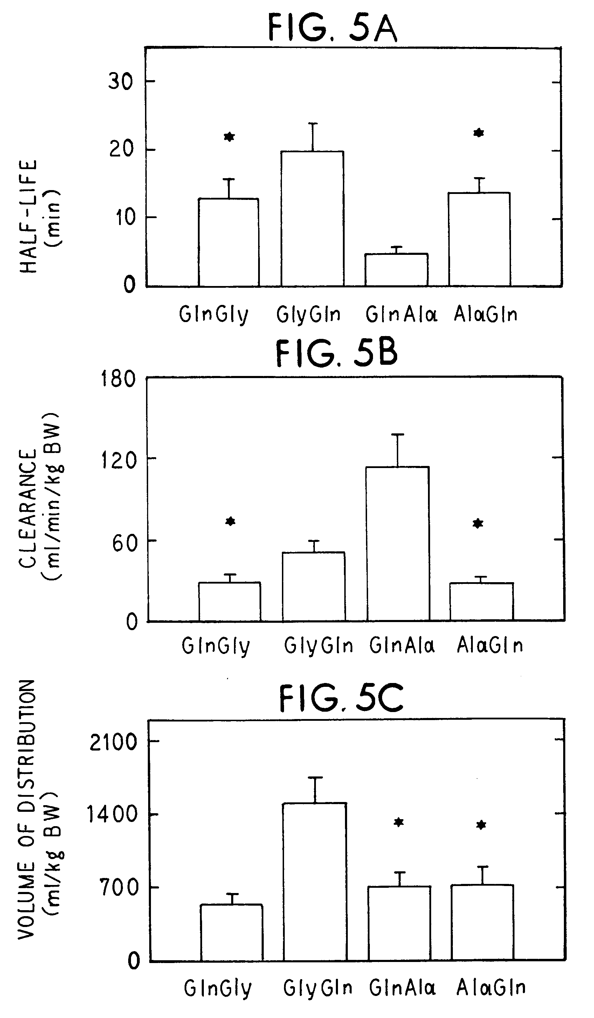

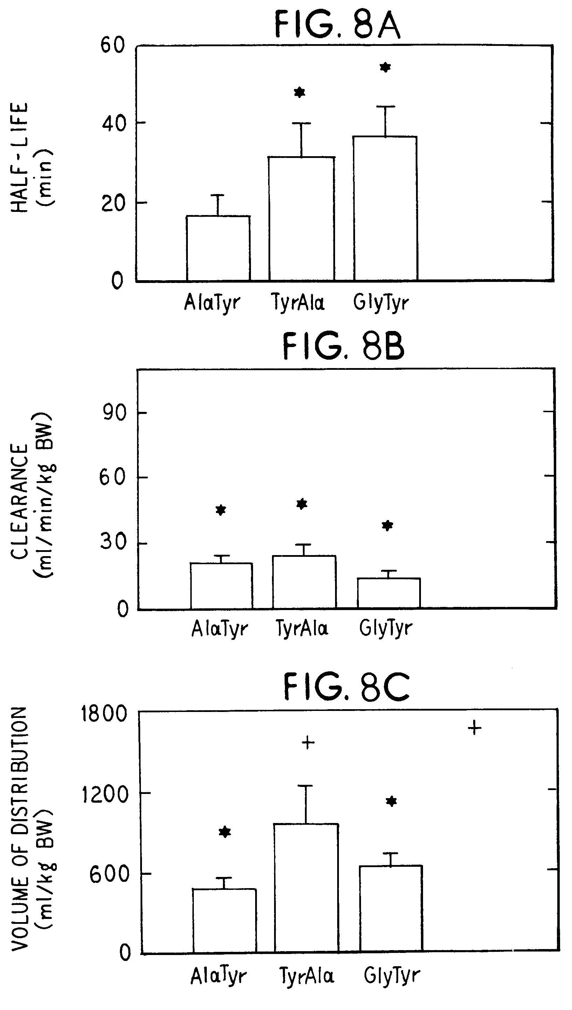

The mean clearance values of the amino acids derived from dipeptides were significantly greater than the clearance values of their parent dipeptides (Table III) (P<0.001 for each dipeptide). For a number of dipeptides (GlnGly, GlyGln, GlnAla and AlaGln) the clearance values of the two amino acids from the same dipeptide were significantly different. While in general the clearance of glutamine and tyrosine was greater than the clearance of glycine and alanine, this difference was not significantly different (ANOVA, P>0.05) due to considerable variability in the clearance of the same amino acid from different dipeptides (see Tables III and IV, and Figures 5-8).

The mean V_{β} of the amino acids (Table IV) derived from the dipeptides was significantly higher than the V_{β} of the parent dipeptides (data not shown) indicating that the amino acids were distributed in a larger volume and/or bound to tissues to a greater extent than the dipeptides. There were significant differences in the mean V_{β} for the two amino acids released from the same dipeptides for GlyTyr, AlaTyr, GlyGln and GlnAla. There was a large variability in the mean V_{β} for the same amino acid arising from different dipeptides (see Table IV and Figures 5-8).

The t_{½} of the amino acids (Table IV) released following dipeptide breakdown was significantly longer than the t_{½} of the parent dipeptides (data not shown) as demonstrated by the slower decline in amino acid concentrations following dipeptide administration (see Figure 1). As was the case for the dipeptides, the t_{½} of the amino acids was generally inversely related to clearance although there were exceptions due to the variation of V_{β} (see Table IV). The t_{½} of the amino acids released from the same dipeptide was significantly different for GlyTyr, AlaTyr, TyrAla, GlnGly and GlnAla.

**TABLE III**

| Peptide Infused | CLEARANCE (mL/min/kg) OF: | | | | |
|---|---|---|---|---|---|
| | Dipeptide | Constituent Amino Acids: | | | |
| | | GLUTAMINE | ALANINE | GLYCINE | TYROSINE |
| GLN-ALA | 278 | 113 | 35 | -- | -- |
| ALA-GLN | 122 | 27 | 43 | -- | -- |
| GLN-GLY | 151 | 29 | -- | 18 | -- |
| GLY-GLN | 43 | 50 | -- | 50 | -- |
| GLY-TYR | 101 | -- | -- | 10 | 13 |
| ALA-TYR | 155 | -- | 21 | -- | 21 |
| TYR-ALA | 187 | -- | 25 | -- | 24 |

Dipeptides were dissolved in saline and infused, via tail vein, into rats (one peptide per animal). Blood samples were taken at timed intervals, measurements were made of concentrations of the peptide and of its constituent amino acids; and pharmacokinetic properties were calculated.

**TABLE IV**

| **Kinetic Parameters of the Amino Acids** | | | | |
|---|---|---|---|---|
| Peptides | Amino Acids | AA Clearance (ml.min⁻¹. kg/BW⁻¹) | V_{β} (ml.kg/BW⁻¹) | t_{½} (min) |
| Gly Tyr | Gly | 10.4 ± 4.33 | 979 ± 164⁺ | 75.4 ± 39.6* |
| | Tyr | 13.1 ± 3.55 | 632 ± 93.0 | 36.4 ± 7.5 |
| Ala Tyr | Ala | 21.4 ± 3.15 | 875 ± 103* | 31.4 ± 8.40⁺ |
| | Tyr | 20.8 ± 3.50 | 475 ± 85.1 | 16.4 ± 5.02 |
| Tyr Ala | Ala | 25.3 ± 4.36 | 853 ± 193 | 20.9 ± 6.30* |
| | Tyr | 23.6 ± 5.20 | 953 ± 283 | 31.0 ± 8.50 |
| Gln Gly | Gln | 29.0 ± 5.15⁺ | 543 ± 102 | 12.8 ± 2.75^{#} |
| | Gly | 18.0 ± 4.91 | 652 ± 121 | 25.1 ± 4.51 |
| Gly Gln | Gln | 50.5 ± 8.31* | 1500 ± 247* | 19.7 ± 4.12 |
| | Gly | 39.3 ± 7.83 | 1150 ± 197 | 21.3 ± 3.31 |
| Gln Ala | Gln | 113 ± 23.8^{#} | 702 ± 135* | 4.54 ± 0.98^{#} |
| | Ala | 35.1 ± 6.14 | 543 ± 79.9 | 10.1 ± 2.15 |
| Ala Gln | Gln | 27.3 ± 4.57⁺ | 723 ± 173 | 13.6 ± 2.14 |
| | Ala | 43.3 ± 7.83 | 569 ± 130 | 13.1 ± 3.95 |
| Note: Values are mean ± S.D. for 5 animals for each dipeptide. t_{½} = terminal half-life and V_{β} = apparent volume of distribution. Differences between constituent amino acids for each dipeptide were assessed using paired t-tests. Significance levels are indicated by: * for P <0.05; ⁺ for P <0.01; and ^{#} for P<0.001. | | | | |

*Comparison of Amino Acid Kinetics from Different Dipeptides (Figure 5-8).*

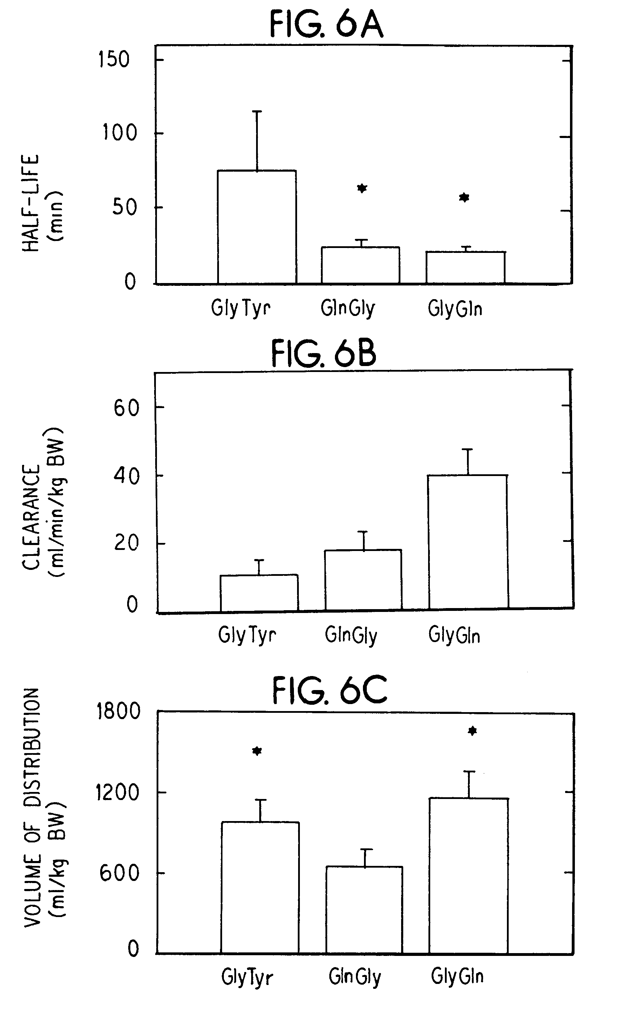

The mean clearance values for glycine were significantly different for all three glycyl-dipeptides (ANOVA, P<0.01) (see Figure 6). The mean clearance of glycine from GlyGln was significantly greater than the clearance of glycine from either GlnGly or GlyTyr.

There was a large range of t_{½} and clearance values for glutamine from the different glutaminyl-dipeptides (see Figure 5); the mean clearance of glutamine from GlnAla was 4 times greater and the mean t_{½} was 3 times shorter than glutamine from GlnGly or AlaGln. Although glutamine from GlyGln had a greater mean clearance than glutamine from GlnGly or AlaGln its mean t_{½} was significantly higher due to its larger mean V_{β}.

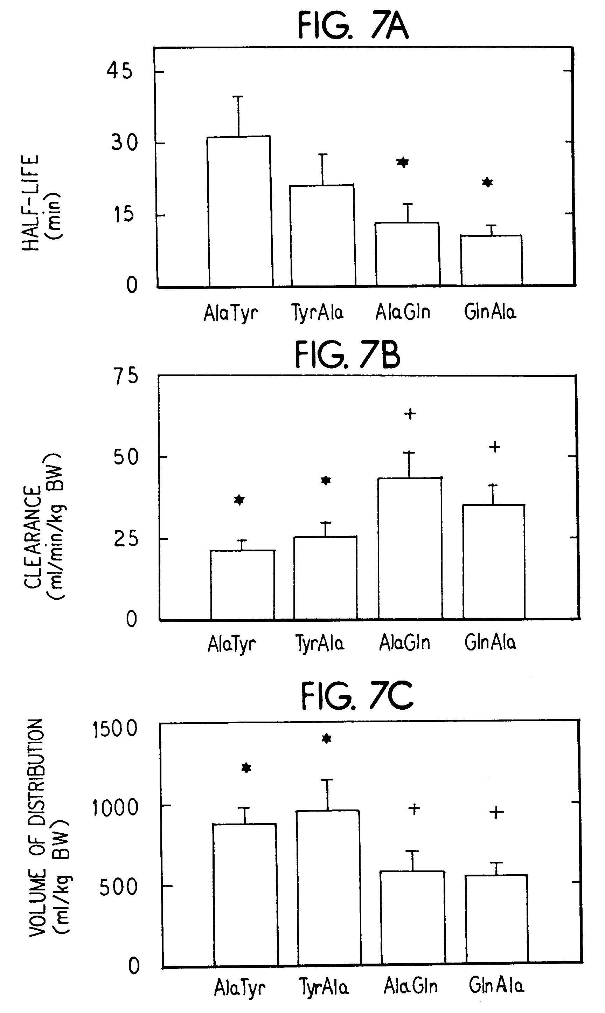

While there was no significant difference in the clearance of tyrosine from AlaTyr and TyrAla the t_{½} of tyrosine from TyrAla was significantly greater than tyrosine from AlaTyr reflecting a greater V_{β} for the former. The t_{½} and V_{β} for alanine from AlaTyr and TyrAla were significantly greater and clearances were significantly lower than alanine from AlaGln and GlnAla (see Figure 7). Although the clearances and V_{β} were not significantly different for alanine from AlaTyr and TyrAla and the t_{½} of alanine from AlaTyr was greater than alanine from TyrAla.

This study represents a detailed investigation into the clearance of dipeptides and their constituent amino acids from blood following bolus intravenous dipeptide injection. Studies in experimental animals and humans using the organ balance technique have shown that the kidneys, liver, muscle and small intestine are the major tissues which clear dipeptides from the blood; 70-100% of dipeptides infused into the blood are removed by these organs. However, tissues vary in their contribution to removal of peptides. In general the kidneys and liver remove the majority (approximately 25% each) of dipeptides entering the blood stream with the small intestine and muscle having smaller roles (each removing approximately 10% of the dose). These studies also show that the rate of dipeptide removal is dependent on structure, with the rate of removal of AlaGln and GlyGln being greater than GlyGln and GlyLeu. Therefore, the greater values of blood clearance of the alanyl-dipeptides reported in this study may reflect their more rapid organ removal.

Uptake of dipeptides across an organ does not necessarily mean actual utilization by tissues of the organ, as there is the possibility of dipeptide hydrolysis in the blood on passage through the organ and, in the case of kidney, excretion in the urine. However, evidence suggests that uptake of dipeptides by the organs does represent assimilation by the tissues.

There are at least two mechanisms of dipeptide utilization by tissues. In liver and muscle, the absence of an active dipeptide transporter suggests that hydrolysis on the extracellular membrane and uptake of the free amino acids is the predominant mechanism of dipeptide assimilation. In contrast, in the kidney and small intestine active dipeptide transporters have been identified in the basolateral membranes.

The fact that V_{β} for many dipeptides was larger than the blood volume of the rats (see Figure 4) suggests that there were some distribution in non-vascular pools. This is consistent with the fact that dipeptides can be either hydrolysed by enzymes on the extracellular surface of cell membranes or taken up into cells and hydrolysed intracellularly. In a similar fashion the considerable variability in V_{β} for the various dipeptides leads to the conclusion that the mechanisms and/or sites of clearance are likely to be different for different dipeptides. This again indicates that the mechanisms of dipeptide metabolism varies between tissues and is at least partly a function of their structure.

Previous studies investigating dipeptide assimilation from the blood have not focussed on the distribution and elimination of the amino acids released from dipeptide breakdown. In the present study the clearances of the amino acids (Table 2) were found to be much greater than the clearances of the parent dipeptides (Figure 3). The utilization of amino acids is thus much slower than dipeptide utilization. Organ dipeptidase studies support these observations, as dipeptidase activity is many times greater than the organ amino acid catabolic activity.

A most significant finding of this study is that the clearance, t_{½} and V_{β} of the amino acids derived from dipeptides are dependent on the structure of the parent dipeptide. The slow fall in amino acid concentration following complete removal of dipeptides from blood (>5 minutes post-injection, Figure 1) indicates either a slow decrease in the release of amino acids from the non-vascular pools and/or slow utilization by the tissues. The fact that the clearance and V_{β} of amino acids varies depending on its parent dipeptide suggests that the amino acids are released into different non-vascular pools where they are either metabolized at different rates or are released at different rates into the blood stream.

The finding that the clearance of the amino acids depends on the dipeptide from which it was cleaved has important implications for intravenous nutrition. Although the blood levels of amino acids during constant infusions of dipeptides would be less than observed immediately following bolus injection, for amino acids such as tyrosine, which is toxic for neonates at high concentrations in the blood, it may be preferable to use a dipeptide such as AlaTyr where the tyrosine moiety is cleared from the blood at a rapid rate.

It should be understood that various changes and modifications to the presently preferred embodiments described herein will be apparent to those skilled in the art. Such changes and modifications can be made without departing from the spirit and scope of the present invention and without diminishing its attendant advantages. It is therefore intended that such changes and modifications be covered by the appended claims.

## Claims

1. Use of peptides of selected structures for the preparation of a composition for providing selected levels of amino acids in the blood of a patient, the peptides providing the desired amino acids to the patient and the structure of the peptides being chosen so as to provide a desired clearance of the amino acids from the blood.

2. Use of peptides of selected structures for the preparation of a composition for parenteral administration for providing amino acids, that may be toxic if present at too great a blood concentration, at a blood concentration below toxic levels, the peptides having a structure that provides for a sufficiently rapid clearance of the amino acids from the patient's blood stream.

3. Use of peptides of selected structures for the preparation of a composition for regulating blood levels of amino acids administered parenterally to a patient, the peptides providing the desired amino acids to the patient and the structure of the peptides being chosen so as to provide a desired clearance of the amino acids from the patient's blood.

4. The use according to any of claims 1 to 3 wherein the peptides are dipeptides.

5. Use of peptides of selected structures for manufacturing a composition for parenteral administering of amino acids to a neonate, the amino acids being provided as dipeptides, the structure of the dipeptides being chosen so as to provide the amino acids so as to insure a sufficiently rapid clearance of the amino acids from the blood of neonates.

6. The use according to claim 5 wherein the dipeptides are administered intravenously.

7. The use according to any of claims 1 to 6 wherein the peptides provide at least one amino acid chosen from glutamine, tyrosine, glycine and phenylalanine.

8. The use according to any of claims 1 to 7 wherein the peptides are chosen from GlyTyr; AlaTyr; TyrAla; GlnGly; GlyGln; GlnAla; and AlaGln.

9. The use according to any of claims 1 to 8 wherein the peptides are administered with other medicaments.
